# EUROPEAN PATENT APPLICATION

(11) **EP 1 589 463 A1**
(43) Date of publication of application: **26.10.2005**
(21) Application number: 04076200.7
(22) Date of filing: 21.04.2004
(51) Int. Cl.: G06F 19/00

(54) **Molecular entity design method**

(71) Applicant: Avantium International B.V., 1014 BV Amsterdam (NL)
(72) Inventor: Brown, Nathan, 2011 VG Haarlem (NL); McKay, Benjamin, 1071 AT Amsterdam (NL)
(74) Representative: Riemens, Roelof Harm

(57) **Abstract**

The invention comprises a method for designing a molecular entity meeting a set of objectives, the method comprising:
a) providing a set of objectives to be met by the molecular entity;
b) providing a population of candidates for the molecular entity;
c) predicting properties of each candidate of the population using at least one empirical model correlating molecular structure to properties or performance measures;
d) scoring the properties or performance measures of each candidate in each objective of the set of objectives;
e) Pareto ranking of the candidates according to the scores for each objective; and
f) selecting at least one of the candidates based on the ranking.

## Description

The invention relates to a method for designing a molecular entity meeting a set of objectives.

Computer-aided molecular design (CAMD) has been an active area of research for a number of years with a substantial amount of this research being directed at evolving novel structures (de novo design) and often-applying genetic search procedures. The most common approaches in this area of CAMD research are the development of fragment-positioning and molecular growth methods for the design of ligand candidates, although constraints on the size and structure of the ligands that are evolved significantly reduces the search space of these problems. However, the research conducted in the area of the more general de novo design of molecules from elements or structural fragments, is less well defined and yet very important in instances when the target is not constrained by a binding pocket, which is the case for more general chemical applications such as considering homogeneous catalysis.

The invention (CoG or Compound Generation) represents a new approach to solving this more general de-novo design problem. CoG evolves novel molecules preferably using a multi-objective graph-based genetic algorithm. The algorithm represents molecules as molecular graphs and operates directly on these graph-based chromosomes using both existing and novel graph-based genetic operators.

Genetic algorithms (GAs) are applied widely in discovering globally optimal solutions to optimisation problem instances, and particularly to problems where no efficient deterministic algorithm is available. The simple GA operates on binary strings, which encode candidate solutions in the search space of interest and perturbs these strings with computational analogues of natural recombination and mutation. Many different configurations of the GA have been applied to solving problems in the field of chemoinformatics.

The genetic programming (GP) algorithm is similar in concept to the GA approach, however the chromosomes are represented as trees rather than the fixed-length strings of the simple GA. The tree representation of GP permits the chromosomes to be both extensible and contractible, through crossover and mutation, a characteristic that is not present in the standard GA, although approaches have been suggested to achieve this.

The tree-based representation of GP is the technique most-often applied for evolving molecular graphs, with two particular approaches being apparent in the method of encoding molecular structures as trees. The first of these approaches generalises molecular fragments as the set of allele values that genes may take. This generalisation obviates the need for complex crossover operators and chromosome repair strategies since cycles are collapsed into single gene nodes in a similar approach to the reduced graph and feature tree techniques. The second approach of representing cyclic graphs as trees uses a special leaf node that points to another node in the graph, basically a hyperlink node, therefore all of the structural information is preserved in the tree allowing the molecule to be expressed as a graph.

Venkatasubramanian, V.; Chan, K.; Caruthers, J. M. Evolutionary Design of Molecules with Desired Properties using the Genetic Algorithm, J. Chem. Inf. Comput. Sci. 1995, 35, 188 -195, discloses a first type of GP approach in evolving novel polymeric structures from a set of molecular fragments. The reported experiments indicate that this approach is very effective at evolving solutions, however the chromosome representation in this work is effectively string-based, or at the very most trees with limited branching.

Nachbar, R. B., Molecular Evolution: Automated Manipulation of Hierarchical Chemical Topology and Its Application to Average Molecular Structures, Genetic Programming and Evolvable Machines 2000, 1, 57 - 94, discloses a second type of GP encoding strategy to the design of novel molecular graphs by encoding the topological structure of molecules as trees. The crossover operator was constrained not to make or break cycles; special mutation operators were defined to control this. This means that any node or edge that is part of a cycle cannot be exchanged in part between chromosomes, considerably restricting the operator. Additionally, Hydrogen atoms are explicitly represented as leaf nodes within the tree. Although this representation allows cyclic graphs to be properly encoded as tree-based chromosomes, the position of the nodes that encode the cycles appears somewhat arbitrary and could easily suffer from side effects from the application of genetic operators. The paper reports that the algorithm has been applied in evolving the average chemical structure of two molecules from their average descriptor vector with the intention of generating a structure that has similar biological activity to both structures.

Although both tree-based representations have been demonstrated to be effective at evolving molecular graphs, they are often limited to either evolving relatively simple types of molecules or, for cyclic structures, employing complex and potentially disruptive genetic operators and repair strategies. It is evident that the molecular graph (or fragment graph) itself can be applied directly as the genotype in a GA approach, although new genetic operators would be required to perturb these types of chromosomes. However, even though the graph itself is intuitively suitable as a chromosome representation, we are aware of only two reported implementations of this method.

US 5 434 796 describes a method of evolving molecules using a genetic search technique. The crossover operator of this approach takes two parents and generates a single child chromosome. However, it has been noted that the crossover operator can result in disconnected graphs, although only in situations where the fitness function can be calculated from disconnected structures. In the crossover operator reported by Weininger, bonds are removed from the parent molecules according to a digestion rate and the resulting fragments are then copied into the child chromosomes according to a dominance rate. The method was reported to be effective at evolving to a given target molecule and for application to novel ligand design using CoMFA (Comparative Molecular Field Analysis).

Globus, A.; Lawton, J.; Wipke, W. T. Automatic Molecular Design Using Evolutionary Algorithms, Nanotechnology 1999, 10, 290 - 299, proposed a graph-based GA to evolve molecular graphs from individual elements. The crossover operator devised by Globus et al. was shown to be very effective at exchanging genetic material between chromosome graphs with relatively minimal disruption to the genetic material and we have adapted this type of crossover operator for CoG.

The encoding of molecular graphs as trees is fraught with issues, requiring either generalised molecular fragments or special node types to implicitly encode cyclic structures. The former approach requires a fragment library to be defined, which may not be possible in all situations. The latter representation complicates crossover operations since apparently simple genetic exchanges will tend to have side effects as a result of gene adjacency not being preserved in the tree. The encoding of any cyclic graphs as trees using this approach will always result in nodes, which are adjacent in the graph, not being adjacent in the tree.

A goal of the invention is to provide a preferably automated design of novel molecular entities with desired properties based on empirical models.

In an embodiment of the invention, this goal is achieved by a method for the multiobjective de *novo* design of novel molecules in *silico* and the application to the Inverse Quantitative Structure Property or Structure Activity Relationships (QSPR / QSAR) and related problems.

Empirical modelling methods such as QSPR / QSAR are widely used to correlate structural variation between molecules to observed differences in their physical or chemical properties. Such relationships can be used to predict the properties or performance of novel compounds. This application is known as virtual screening.

However, a satisfactory solution for the inverse problem, generating a molecular structure with desired property values based on QSPR / QSAR models has been previously unavailable. This invention (CoG) proposes a solution to this inverse problem.

This problem is commercially important, as it would allow the automated design of, for instance, novel homogeneous catalysts, chemical reagents, or formulation additives for polymers, fuels and oils with desired properties, based on limited screening results.

For solving the inverse QSAR / QSPR problem, CoG combines the flowing elements:
- A genetic algorithm (GA).
- A graph-based representation of molecules.
- A multiple objective fitness function utilising Pareto ranking.
- A number of empirical models (QSPR / QSAR's) describing the properties of interest.
Each of these elements is discussed in more detail below:
Genetic algorithm
Genetic algorithms (GAs) are applied widely in discovering globally optimal solutions to optimisation problems, particularly where no efficient deterministic algorithm is available. In essence, a GA takes a "population" of potential solutions (e.g. encoded molecular structures) to a problem (e.g. inverse QSPR) and "evolves" them by repeatedly applying a variety of computational analogues to biological crossover and mutation. Solutions are preferentially selected for "breeding" based on how well they solve the specified problem. Over a number of "generations" the quality of the candidate solutions improves until an acceptable solution is obtained. The basic GA is described in Goldberg, D. E. Genetic Algorithms in Search, Optimisation and Machine Learning; Addison-Wesley: Reading, MA, 1989.

A genetic algorithm is a component of the current invention. No efficient deterministic algorithm is available for searching chemical space. While there are many varieties of GA, in the following sections, the importance of a graph-based and multi-objective GA is outlined.

### Graph-based representation of molecules

In chemoinformatics, GA's have been widely used for solving various problems. Molecules have been represented as binary strings, trees or graphs.
CoG adopts a graph-based representation of molecules. The advantages of this graph-based representation are as follows:
- Molecular structures are more simply and transparently represented as graphs (e.g. where atoms are nodes and bonds are edges) than more abstract representations such as bit-strings or trees.
- This allows more effective exchange of information between potential solutions during "crossover", with less disruption than bit-string or tree-based representations.
- A graph-based encoding also facilitates the representation of molecular fragments, rather than atoms, as nodes of the graph. This provides a convenient way of including prior knowledge or restricting the search space.
While bit-string or tree based GA's can be successfully applied to solving the inverse QSPR / QSAR problem (in some application domains), it is likely that such an approach would forgo the advantages outlined above.

Multiple objective fitness function utilising Pareto ranking

There are typically multiple criteria to consider when designing a novel molecule. For example, what are its boiling point, melting point, solubility and chemical reactivity? It is therefore advantageous to incorporate these criteria as multiple objectives in any inverse QSPR /QSAR algorithm.

For this purpose, GoG utilizes a multiple objective genetic algorithm (MOGA). The MOGA approach uses Pareto ranking to grade the relative performance of potential solutions (i.e. molecules). The Pareto method ranks solutions according to the number of other solutions that outperform them in all of the objectives being considered (i.e. dominate them). In chemoinformatics, Pareto ranking has been applied to a number of multiobjective optimisation problems with significant success.

The most common alternative to Pareto ranking for multiple objective optimisation is to determine the fitness of a potential solution by taking a simple "weighted average" of performance with respect to each of the objectives. This approach is less satisfactory than Pareto ranking as it requires the user to judge the relative importance (and relative difficulty) of each objective a-priori. In addition, it allows performance in one objective to be sacrificed for good performance in another. In comparison, Pareto ranking finds a wide range of non-dominated solutions to the posed problem and allows the user to make the final design trade-off.

While it is possible for the "weighted average" approach to multi-objective optimisation to be applied to the inverse QSPR / QSAR problem, it would forgo the advantage outlined above.

### QSPR / QSAR' s

A great deal has been published on Quantitative Structure Property Relationship / Quantity Structure Activity Relationships (QSPR / QSAR's) and their application to virtual screening. In essence, QSPR / QSAR's are empirical models that correlate differences in a molecules structure to observed differences in the physical, chemical / biological properties thereof. These models are typically generated using statistical methods such as Partial Least Squares (PLS) or neural networks.

The combination of QSPR and GA's for solving the inverse QSPR problem has been reported in the literature. However, to our knowledge, never in combination with a graph-based representation or a multi-objective approach. Instead of or in addition to QSPR and/or QSAR, a Quantitative Structure Toxicity Relationship, a Quantitative Structure Reactivity Relationship and/or a Quantitative Structure Selectivity Relationship can be applied.

In other words, the invention can be defined as a method for designing a molecular entity meeting a set of objectives, the method comprising:
a) providing a set of objectives to be met by the molecular entity;
b) providing a population of candidates for the molecular entity;
c) predicting properties of each candidate of the population using at least one empirical model correlating molecular structure to properties or perfromance measures;
d) scoring the properties or performance of each candidate in each objective of the set of objectives;
e) Pareto ranking of the candidates according to the scores for each objective;
f) selecting at least one of the candidates based on the ranking.

A molecular entity can comprise any kind of molecule such as organic molecules such as proteins, carbohydrates, nucleic acids, polymers, molecules having biological action such as pharmaceuticals, enzymes, however inorganic molecules such as catalysts, are also encompassed by the invention. Further, parts of molecules, in particular of biological molecules, such as domains having enzymatic activity, are also encompassed by the invention. The objectives to be met by the molecular entity may comprise any kind of desired property, such as a physical property (e.g. a melting point, boiling point, polarity, solubility, etc.), chemical, or biological properties (such as reactivity, toxicity, selectivity, etc.). In addition to, or instead of the objectives as mentioned, any other suitable objective might be used. A population of candidates i.e. a plurality of candidates which might be suitable for meeting the objective or which might comprise molecule parts potentially suitable for meeting one or more of the objectives. The skilled person, based on general knowledge in the particular field will be able to provide a suitable population of candidates, the particular objectives being provided. Using the model, the properties of each candidate are predicted. Then, the properties or performance of each candidate is scored preferably for each objective of the set of objectives. Then, the scores for each candidate are ranked according to a Pareto ranking and based on the ranking at least one candidate is selected. Should the candidate meet the objectives, then no further actions are required, however in case that the selected candidate does not yet meet the objectives or does not sufficiently well meets the objectives, a genetic approach is preferably followed by selecting at least two candidates in step f) and g) creating a new population of candidates by perturbing the selected candidates; and h) repeating steps c), d), e) and f) for the new population of candidates. This process can be repeated as often as required, i.e. the steps g), c), d), e) and f) are repeated for each new population. Such repeating can be performed a predetermined number of cycles and/or until a candidate has been selected which meets the set of objectives. In this document, perturbing is to be understood as creating new molecular structures by making modifications to (representations of) candidate molecules or combining structural information from (representations of) multiple candidate molecules. The perturbation can comprise cross over, mutation and/or reproduction.

The candidates for the molecular entity can be represented with a graph based representation, advantages thereof having been explained above.

The invention will now be described referring to the drawing showing a non limiting embodiment of the invention, in which:

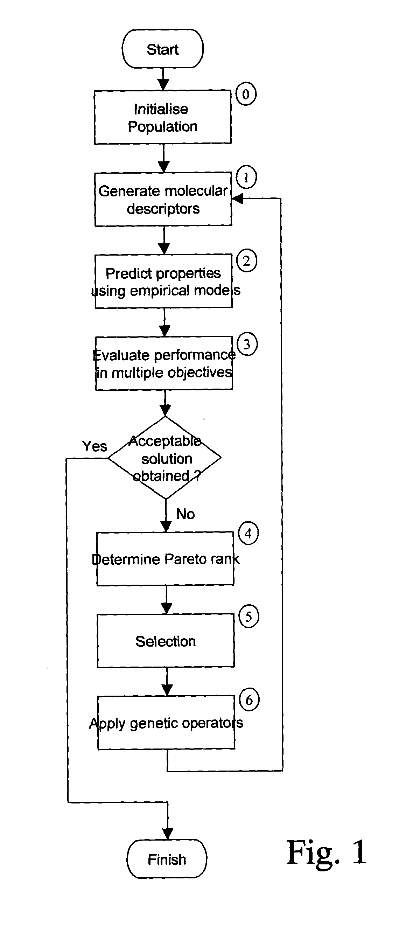

Fig. 1 depicts a flow diagram of an embodiment of the method according to the invention.

The algorithm as depicted in fig. 1 comprises a series of computational steps:
0. Initialise a population of "individual" potential solutions (candidates)
1. Generate molecular descriptors for each individual
2. Predict a number of properties for each individual using one or more QSPR / QSAR models
3. Score each individuals relative performance in multiple objectives
4. Pareto rank individuals according to their scores on all objectives.
5. For each new generation repeatedly select "parent" individuals with a bias towards "better" individuals
6. Create "children" (a new population of candidates) by applying genetic operators to the "parents":
   - Crossover: children are created by recombining parts of two parents.
   - Mutation: a single parent is modified to create a child.
   - Reproduction: a single parent is copied into the new generation.
Repeat steps 1 to 6 until a solution (i.e. molecule) with the desired performance is "discovered".

The above mentioned steps will now be described in more detail.
Step 1: in this workflow advantageously applies to our proprietary Fingal molecular fingerprinting algorithm for calculating similarity metrics from molecular graphs. However, any molecular descriptor generation method(s) could feasibly replace this algorithm.
Step 2 in this workflow refers to QSPR / QSAR models but could conceivably incorporate Quantitative Structure Toxicity /Reactivity QSTR / QSRR models and other related methods. These models can be developed using any statistical or machine-learning method (e.g. PLS, neural nets, regression trees) that can build empirical models from the descriptors calculated in step 1. In the proposed invention, the model output also includes an indication of distance from the model (e.g. Hotelling T² or DmodX) or prediction performance.
Step 3: The model outputs obtained in Step 3 are evaluated relative to a number of objectives that have been defined by the user. For the inverse QSPR problem, the multiple objectives may include a number of physical property targets or ranges (e.g. melting point < 50 deg C, maximum aqueous solubility and maximum chemical reactivity) and an indication of a molecules "membership" of the models used to predict these properties. The later is a novel development that keeps the algorithm from extrapolating beyond the valid range of the empirical models. This approach is only feasible because of the adopted MOGA framework.
Step 4 applies the Pareto ranking method to order the candidate solutions according to dominance in the objectives. This is believed to be a novel application of Pareto ranking for the direct evolution of structure.
Step 5 refers to selection of potential solutions from the population for the application of genetic operators (refer to Step 6) to generate the next generation of solutions. Solutions that have a low Pareto ranking (i.e. have been relatively successful at meeting the objectives) are more likely to be selected. The CoG algorithm currently uses the common GA selection method known as tournament selection. However, any of the commonly used GA selection methods would also be acceptable.
Step 6: A number of "genetic operators" are applied to the selected solutions to generate new potential solutions. The adopted graph-based representation requires the implementation of specialised genetic operators. In CoG we have, where possible, adapted genetic operator from the literature. We have also developed a number of novel genetic operators.
• Mutation: Node mutation (append, prune, insert and delete) edge mutation (add, delete and substitution)
• Crossover: Multi-point crossover. Subgraph crossover.

The method according to the invention thus provides an automatic suggestion of novel molecular entities that conform to specified responses of interest.

## Claims

1. A method for designing a molecular entity meeting a set of objectives, the method comprising:
a) providing a set of objectives to be met by the molecular entity;
b) providing a population of candidates for the molecular entity;
c) predicting properties of each candidate of the population using at least one empirical model correlating molecular structure to properties or performance measures;
d) scoring the properties or performance of each candidate in each objective of the set of objectives;
e) Pareto ranking of the candidates according to the scores for each objective; and
f) selecting at least one of the candidates based on the ranking.

2. The method according to claim 1, wherein at least two candidates are selected in step f), the method further comprising:
g) creating a new population of candidates by perturbing the selected candidates; and
h) repeating steps c), d), e) and f) for the new population of candidates.

3. The method according to claim 1 or 2, wherein a graph based representation is used for representing the candidates for the molecular entity.

4. The method according to any of the preceding claims, wherein the model comprises a Quantitative Structure Property Relationship, a Quantitative Structure Activity Relationship, a Quantitative Structure Toxicity Relationship, a Quantitative Structure Reactivity Relationship, a Quantitative Structure Selectivity Relationship or any combination thereof.
